# EUROPEAN PATENT SPECIFICATION

(11) **EP 0 242 984 B1**
(45) Date of publication and mention of the grant of the patent: **10.03.1993**
(21) Application number: 87302363.4
(22) Date of filing: 19.03.1987
(51) Int. Cl.: C12M 3/00

(54) **Culture device**
Kultureinrichtung
Appareil de culture

(30) Priority: 21.03.1986 GB 8607046
(43) Date of publication of application: 28.10.1987
(73) Proprietor: SANYO GALLENKAMP PLC, London EC4A 2NT (GB)
(72) Inventor: Macleod, Alexander James, 21 Ellens Glen Road, Edinburgh, Scotland (GB); Foster, Peter Reynolds, 21 Ellens Glen Road, Edinburgh, Scotland (GB)
(74) Representative: Milhench, Howard Leslie

(56) References cited:
- FR-A- 2 324 365
- GB-A- 1 238 401
- US-A- 4 178 209

## Description

This invention relates to a fermenter and a method for the culture and/or separation of cells.

A wide variety of cellular organisms are cultured for industrial or other purposes. Culturing of mammalian cells is of particular interest, eg in the production of monoclonal antibodies. Continuous culturing processes are advantageous since they enhance the productivity of the fermenter and reduce the amount of time required for cleaning of the apparatus.

Continuous fermenters, that is fermenters in which nutrient is supplied to the fermenter during the course of the culturing process, are known but they suffer from a number of disadvantages. Gassification of culture medium usually produces a gas-liquid interface, eg in air-lift fermenters such as that described in International Patent Application No WO 86/07605. Such an interface can be detrimental to cultured cells because a cell may be stretched across the interface causing damage to the cell.

Other continuous fermenters provide for gassification of the nutrient remote from the culture followed by continuous perfusion of the nutrient through the culture, eg spin-filter fermenters such as those described by S Renveny et al, J. Immunological Methods, 1986, 86, 61-69. Spin-filter fermenters have the disadvantage that dead-cells accumulate in the culture which reduces the productivity of the fermenter. A further disadvantage of spin-filter fermenters is that the filter fouls easily thus restricting the steady flow of culture medium. Although methods for reducing fouling of the filter are known (e.g. WO 86/07605) they include the undesirable incorporation of a gas-liquid interface.

French Patent No. 2,324,365 describes an apparatus for continuous cultivation of microorganisms which comprises a relatively small chamber in which the cells are cultured. The chamber is defined intermediate two water swelled membranes which in use, are pressed onto a helical stirrer which rotates therebetween.

The present invention seeks to provide a fermenter and a method for the continuous culture of cells which removes the requirement for a gas-liquid interface, minimises the generation of the shear conditions in the culture thereby improving cell viability and reduces fouling of the filter.

Accordingly, the present invention provides a fermenter for continuous cell culture comprising a nutrient inlet, an outer chamber for receiving nutrient and an inner chamber for growing cells in culture and which is at least partially enclosed within the outer chamber, the inner chamber being defined by a wall at least a portion of which is semi-permeable, the fermenter being characterised in that the inner chamber contains a helical ribbon stirrer spaced from the inner chamber wall and which is arranged to prevent fouling of the semi-permeable portion of the inner chamber wall by causing local agitation of culture adjacent the semi-permeable wall whilst minimizing the generation of shear forces.

The term semi-permeable means permeable to nutrient but impermeable to the culture.

The helical ribbon stirrer acts indirectly on the wall by causing local agitation of the culture immediately adjacent the semi-permeable wall thereby preventing fouling of the semi-permeable wall.

In view of the sensitivity of cells to degradation by shear forces the helical ribbon stirrer provides maximum cleansing of the semi-permeable wall whilst providing minimum shear forces which may cause damage to cells. Preferably a multi-helical ribbon stirrer is used. Multi-helical stirrers are known (Bourne, et al Trans. Instn. Chem. Engrs. Vol 47, 1969) but are new to the field of continuous fermentation. The number of blades of the multi-helical ribbon stirrer is preferably from 1 to 10, more preferably from 1 to 4 and most preferably 1 or 2. A blade angle of 5 to 85° to the semi-permeable wall is preferred, more preferably 30 to 60° and most preferably 40 to 50°.

The speed of the stirrer is preferably from 10 to 250 revolutions per minute (rpm), preferably 50 to 200 rpm and most preferably 75 to 150 rpm.

The separation between the stirrer and the surface of the semi-permeable wall is preferably as small as possible whilst avoiding damage to the semi-permeable wall. It is the turbulence adjacent the semi-permeable wall, created by the stirrer, which actually facilitates the prevention of fouling. This will of course be dependent upon the path of the stirrer relative to the semi-permeable wall and the size of cells which are cultured. In particular it is dependent upon the separation of the stirrer and the semi-permeable wall. The separation is preferably from 1 to 10mm, more preferably from 1 to 5mm and most preferably from 1 to 2mm.

In order to further reduce fouling of the semi-permeable wall the inner chamber is preferably of a shape which permits the use of low flow rates of nutrient. Therefore preferably the inner chamber is of a shape which has a large surface area, eg cylindrical in which the side walls are semi-permeable and most preferably both the inner, growing, chamber and the outer nutrient chamber are cylindrical in shape.

Preferably the whole of the inner growing chamber is enclosed by the outer chamber.

The inner growing chamber may also be provided with means for removing cell culture without stopping the continuous fermentation process. The means for removing cell culture may comprise a pump, eg a peristaltic pump, or if the vessel is operating under positive pressure it may utilise that pressure. The means may comprise a combination of the two. The removal of portions of the cell culture is also advantageous because systems in which cell culture cannot be removed during the course of the fermentation process will eventually be self-terminating as the cells multiply beyond their concentration limit, degenerate and die. Furthermore, the fermenter may also be provided with means for removal of nutrient. The fermenter preferably comprises both means for removal of culture and nutrient.

The wall separating the inner and outer chambers may comprise only a portion which is semi-permeable. However preferably the whole of the wall is semi-permeable. The semi-permeable wall may be, for example, a membrane, a glass sinter, a stainless steel mesh or a membrane supported on a stainless steel mesh. The semi-permeable wall is preferably sufficiently rigid to retain a cylindrical shape without the requirement for a support. Most particularly the semi-permeable wall must be resistant to sterilisation, eg with steam. The pore size will be determined inter alia by the type of cell to be cultured. Therefore, for mammalian cells the pore size is preferably from 1 to 10 micrometers, more preferably from 1 to 5 micrometers, and most preferably from 3 to 5 micrometers.

The cells may, if desired, be cultured as an adherent layer on a carrier, e.g. beads of conventional type, such as a dextran. Alternatively and preferably, the cells may be cultured as a free suspension in the culture medium. The fermenter may be used for the culture of any cells capable of in vitro growth in suspension liquid culture (including microcarrier culture), but is especially useful for the culture of animal cells, eg hybridoma cells.

The space between the inner and outer chambers is preferably as small as possible consistent with a free flow of culture medium. It is however desirable to avoid dead spaces in this space and it may be desirable to provide a purge, or medium bypass, so that such dead spaces do not occur.

The nutrient may flow through an inlet, into the outer chamber and, through an outlet, out of the outer chamber. However, it is prefered that the nutrient flow directly into the inner growing chamber, through the semi-permeable wall and then out of the outer nutrient chamber.

The outer chamber may therefore be provided with a nutrient inlet and a nutrient outlet. Preferably only the inner growing chamber is provided with a nutrient inlet and only the outer nutrient chamber with a nutrient outlet.

The fermenter may be provided with means for heating the culture medium supplied to the device and means, for example, including thermal insulation, for controlling the temperature of the culture medium. It may also be provided with means for measuring various parameters, e.g. temperature, dissolved oxygen p(O₂), pH, redox potential etc, in one or both of the inlet and outlet streams for the nutrient. Such means which are not in contact with the cell culture are, however, less liable to fouling, than are the equivalent sensors which are in direct contact with the culture.

The device according to the invention may be used in conjunction with means for providing appropriate nutrient, recycling, analysis, conditioning, oxygenating and topping up of the nutrient.

Also, provided is a method for the continuous culture of cells using a fermenter described above, the method comprising:
(a) placing a cell suspension in the inner chamber;
(b) continuously supplying nutrient to the inner chamber via the nutrient inlet; and
(c) stirring the cell culture with the helical ribbon stirrer to prevent fouling of the semi-permeable wall, such stirring causing agitation of the cell suspension immediately adjacent to the semi-permeable wall whilst minimising the generation of shear forces such as might cause cell rupture.

The present method has advantages over conventional continuous culture methods. It is known that when certain mammalian cells are under stress they produce Epstein-Barr Virus (EBV). Therefore by avoiding stressful conditions EBV may be eliminated. Furthermore, conventional methods suffer from the disadvantage of foaming due to the presence of proteins in the nutrient which foam readily on aeration. Foam is particularly undesirable since it provides a large number of gas-liquid interfaces. Therefore by keeping the cells separate from the nutrient at the point of aeration they are not damaged by the foam. Furthermore, separation of the cells at the point of aeration avoids exposure of the cells to shear forces whilst the nutrient is aerated. By removing conditions which are stressful to cells, eg gas-liquid interface and excessive shear forces; cell damage is reduced, and the fermenter may be operated in the absence of anti-foaming agents.

Therefore, the present method for the continuous culture of cells as hereinbefore described can be carried out in the absence of an anti-foaming agent.

The fermenter and method of the invention are advantageous because fewer dead cells are produced in the culture and fouling of the wall between the inner and outer chamber is greatly reduced. We have found that the fermenter is particularly useful in the production of antibody containing solutions, especially monoclonal antibodies. Thus by culturing monoclonal antibody secreting hybridoma cell lines using a fermenter according to the invention it is possible to prepare antibody containing solutions which have a low level of contamination from dead cells and cellular debris. These solutions tend to have a high antibody purity. Thus such solutions can be used either directly in non-clinical studies, eg in diagnostic immuno assays, or may be readily isolated and purified further, eg for clinical use. We have found that it is possible to prepare solutions of antibody of greater than 30% purity, eg 50% purity, calculated on the basis of total soluble proteins present in solution.

Accordingly, an antibody containing solution can be prepared by the above method.

For the preparation of clinical quality antibodies a purification step may be added. Any conventional purification techniques may be used, eg ion-exchange chromatography.

In particular the fermenter is advantageous because it is capable of maximising productivity by restricting all stress as hereinbefore described. Production rate is dependent upon the particular cell line involved, however in continuous operation the fermenter may produce monoclonal antibodies at a rate of greater than 0.5g McAb/l/d, preferably greater than 0.75g McAb/l/d and most preferably greater than 1.0g McAb/l/d.

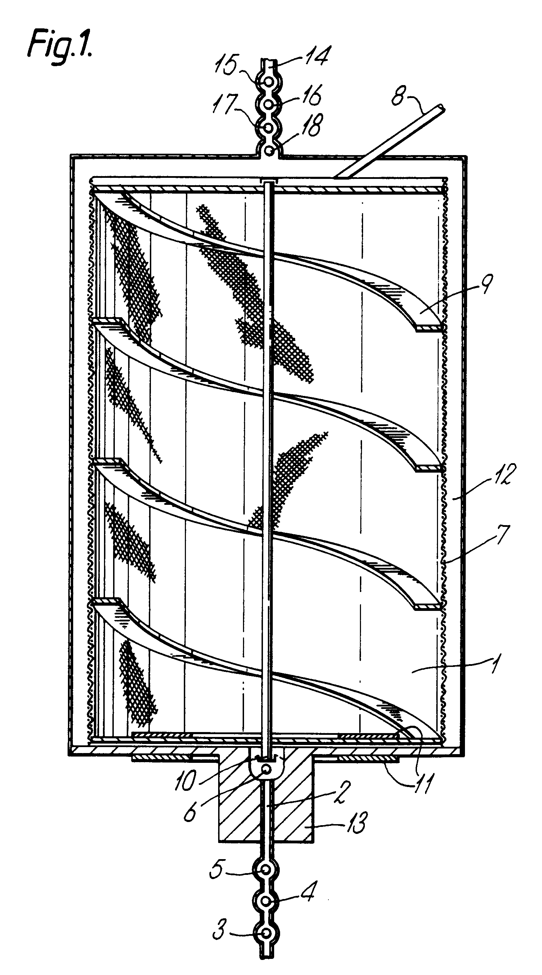

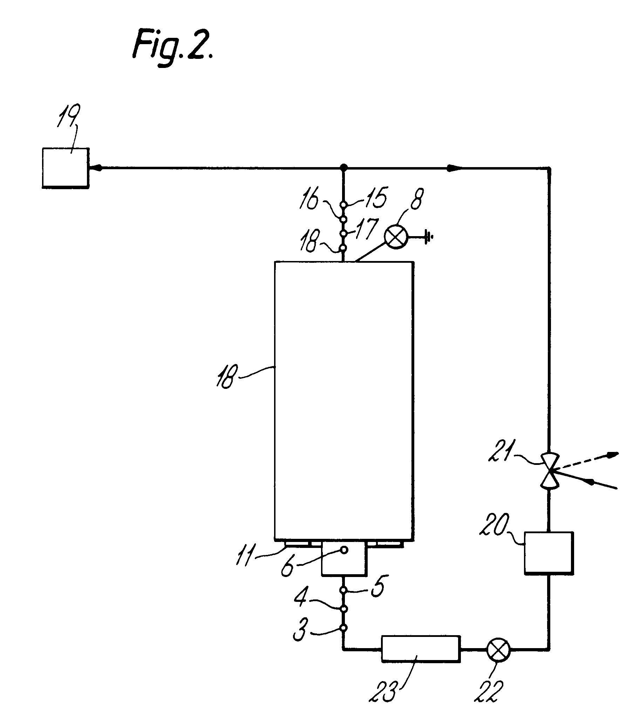

The invention is illustrated by the attached drawings which are not to scale in which Figure 1 represents a vertical section through a device according to the invention and Figure 2 shows schematically a system incorporating such a device.

In Figure 1 the fermenter comprises an inner cylindrical chamber (1), e.g. of from 5 to 100L capacity enclosed by an outer cylindrical chamber (12). The base of the inner chamber (1) is provided with a nutrient inlet (2) in which there are situated sensors for redox potential (3), pH (4), p O₂ (5) and temperature (6). The inner chamber (1) has outer sintered stainless steel mesh side walls (7) and an upper outlet (8) for removal of a proportion of the cultured cells. Within the inner chamber (1) is a double helical ribbon stirrer (9) which is rotatably mounted on a pivot (10) located in the base of the outer chamber (12) and which is capable of being driven by magnetic coupling (11), or by other suitable means. The blades of the stirrer (9) are adapted to sweep very close, eg within 1mm, of the inner side wall of the inner chamber (1).

The nutrient inlet (2) is provided with a concentrically mounted heater (13), to ensure that nutrient enters the fermenter at the desired temperature. A nutrient outlet (14) is provided in the roof of the outer chamber (12). Sensors for redox potential (15), pH (16), p O₂ (17) and temperature (18) are located in the flow path of outlet (14).

In operation an initial inoculum of the cells to be cultured, eg hybridoma cells, synthesising monoclonal antibodies, and the culture medium, eg RPMI 1640, are introduced into inner chamber (1) which has, eg a 5 litre capacity. When appropriate culture conditions have been established within chamber (1), for example pH of from 7.1 to 7.3, temperature of from 36 to 37.5°C, pO₂ of from 80 to 100% saturation with air at 1 atmosphere, nutrient is passed through inlet (2). The flow rate of the nutrient may be from 0.5 to 5.0 litres per minute. The nutrient passes through chamber (1) and into the space between chambers (1) and (12) through the outer walls (7). In so doing cultured cells are drawn towards the inner surface of the outer walls (7), but are prevented from collecting on the walls by rotation of the double helical ribbon stirrer (9). A proportion (which will depend on the particular cells being cultured and the culture conditions to be used) of the culture may be removed from inner chamber (1) by means of outlet (8), eg 2.5 litres in, for example 12 to 24 hours.

Figure 2 shows the culture unit 18 connected to an analysis/sampling unit (19), a medium conditioning unit (20), a valve (21) to enable fresh nutrient to be added and for a proportion of the recycling nutrient to be removed, a pump (22) and an oxygen exchange unit (23).

## Claims

1. A fermenter for continuous cell culture comprising a nutrient inlet, an outer chamber for receiving nutrient and an inner chamber for growing cells in culture and which is at least partially enclosed within the outer chamber, the inner chamber being defined by a wall at least a portion of which is semi-permeable, the fermenter being characterised in that the inner chamber contains a helical ribbon stirrer spaced from the inner chamber wall and which is arranged to prevent fouling of the semi-permeable portion of the inner chamber wall by causing local agitation of culture adjacent the semi-permeable wall whilst minimizing the generation of shear forces.

2. A fermenter according to claim 1, wherein the helical ribbon stirrer comprises from 1 to 10 blades.

3. A fermenter according to claim 1 or claim 2, wherein the spacing between the helical ribbon stirrer and the semi-permeable wall is from 1 to 10mm.

4. A fermenter according to any one of claims 1 to 3, wherein the inner chamber is cylindrical.

5. A fermenter according to any one of claims 1 to 4, wherein the inner chamber is provided with means for removing cell culture without stopping the continuous fermentation process.

6. A method of growing cells continuously in culture in a fermenter according to any preceding claim the method comprising:
(a) placing a cell suspension in the inner chamber;
(b) continuously supplying nutrient to the inner chamber via the nutrient inlet; and
(c) stirring the cell culture with the helical ribbon stirrer to prevent fouling of the semi-permeable wall, such stirring causing agitation of the cell suspension immediately adjacent to the semi-permeable wall whilst minimising the generation of shear forces such as might cause cell rupture.

7. A method according to claim 6, wherein the continuous culture is carried out in the absence of an anti-foaming agent.

8. A method according to claim 6 or claim 7, further comprising removing at least a portion of the cell suspension from the inner chamber when the cell density exceeds a predetermined level.

9. A method of preparing an antibody solution comprising culturing antibody-secreting cells by a method according to any of claims 6 to 8.

## Patentansprüche

1. Fermentierer zur stetigen Zellzucht mit einem Nährstoffeinlaß,
einer äußeren Kammer für die Aufnahme von Nährstoff und einer inneren Kammer für die Aufzucht von Zellkulturen, die zumindest teilweise innerhalb der äußeren Kammer umschlossen ist, wobei die innere Kammer durch eine Wand ausgebildet wird, von der zumindest ein Abschnitt halb-durchlässig ist, wobei der Fermentierer **dadurch gekennzeichnet ist, daß**
die innere Kammer einen Spiralbandrührer beinhaltet, der von der inneren Kammerwandung beabstandet und dafür vorgesehen ist, durch Auslösen einer lokalen Agitation bzw. heftigen Bewegung der an die halb-durchlässige Wand angrenzenden Kultur bei Minimieren der Erzeugung von Scherkräften ein Bewuchs des halb-durchlässigen Abschnitts der inneren Kammerwand zu verhindern.

2. Fermentierer nach Anspruch 1, **dadurch gekennzeichnet, daß**
der Spiralbandrührer 1 bis 10 Blätter aufweist.

3. Fermentierer nach Anspruch 1 oder 2, **dadurch gekennzeichnet, daß**
der Abstand Zwischen dem Spiralbandrührer und der halb-durchlässigen Wand 1 bis 10 mm beträgt.

4. Fermentierer nach einem der Ansprüche 1 bis 3, **dadurch gekennzeichnet, daß**
die innere Kammer zylindrisch ist.

5. Fermentierer nach einem der Ansprüche 1 bis 4, **dadurch gekennzeichnet, daß**
die innere Kammer mit einer Einrichung für das Entfernen der Zellkultur ohne Stoppen des stetigen Fermentierprozesses versehen ist.

6. Verfahren zur stetigen Zucht von Zellen in Kulturen in einem Fermentierer entsprechend einem vorstehenden Anspruch, wobei das Verfahren folgende Schritte aufweist:
(a) Hineingeben einer Zellsuspension in die innere Kammer;
(b) stetiges Zuführen von Nährstoff in die innere Kammer über den Nährstoffeinlaß, und
(c) Rühren der Zellkultur mit dem Spiralbandrührer, um ein Bewuchs der halb-durchlässigen Wand zu verhindern, wobei ein derartiges Rühren eine Agitation der unmittelbar an die halb-durchlässige Wand angrenzenden Zellsuspension verursacht, während die Erzeugung von denjenigen Scherkräften, welche ein Zerplatzen der Zellen verursachen könnten, minimiert wird.

7. Verfahren nach Anspruch 6, **dadurch gekennzeichnet, daß**
die stetige Zucht in Abwesenheit eines Antischäum-Mittels ausgeführt wird.

8. Verfahren nach Anspruch 6 oder 7 **gekennzeichnet durch den weiteren Schritt:**
Entfernen zumindest eines Teils der Zellsuspension aus der inneren Kammer wenn die Zelldichte ein vorbestimmtes Niveau übersteigt.

9. Verfahren für das Zubereiten einer Antikörperlösung **gekennzeichnet durch** den folgenden Schritt:
Züchten von Antikörper-ausscheidenden Zellen durch ein Verfahren nach einem der Ansprüche 6 bis 8.

## Revendications

1. Cuve de fermentation pour la culture de cellules en continu, comportant un orifice d'entrée de substance nutritive, une chambre extérieure destinée à recevoir une substance nutritive et une chambre intérieure destinée à développer des cellules en culture et au moins partiellement enfermée à l'intérieur de la chambre extérieure, la chambre intérieure étant définie par une paroi dont une partie au moins est semi-perméable, la cuve de fermentation étant caractérisée en ce que la chambre intérieure contient un agitateur à ruban en spirale espacé de la paroi de la chambre intérieure et conçu pour empêcher un encrassement de la partie semi-perméable de la paroi de la chambre intérieure en provoquant une agitation locale de la culture à proximité de la paroi semi-perméable tout en réduisant au minimum la production de forces de cisaillement.

2. Cuve de fermentation selon la revendication 1, dans laquelle l'agitateur à ruban en spirale comporte de 1 à 10 ailettes.

3. Cuve de fermentation selon la revendication 1 ou 2, dans laquelle l'espacement entre l'agitateur à ruban en spirale et la paroi semi-perméable est de 1 à 10 mm.

4. Cuve de fermentation selon l'une quelconque des revendications 1 à 3, dans laquelle la chambre intérieure est cylindrique.

5. Cuve de fermentation selon l'une quelconque des revendications 1 à 4, dans laquelle la chambre intérieure est pourvue de moyens pour extraire de la culture de cellules sans interrompre le processus de fermentation continu.

6. Méthode pour développer en continu dos cellules en culture dans une cuve de fermentation selon l'une quelconque des revendications précédentes, la méthode consistant à :
(a) placer une suspension de cellules dans la chambre intérieure;
(b) délivrer en continu une substance nutritive à la chambre intérieure par l'intermédiaire de l'orifice d'entrée de substance nutritive; et
(c) agiter la culture de cellules à l'aide d'un agitateur à ruban en spirale pour empêcher un encrassement de la paroi semi-perméable, cette agitation provoquant une agitation de la suspension de cellules au voisinage immédiat de la paroi semi-perméable tout en réduisant un minimum la production de forces de cisaillement susceptibles d'entraîner une rupture des cellules.

7. Méthode selon la revendication 6, dans laquelle la culture en continu est réalisée en l'absence d'un agent anti-moussant.

8. Méthode selon la revendication 6 ou 7, consistant également à extraire de la chambre intérieure au moins une partie de la suspension de cellules, lorsque la densité des cellules excède un niveau prédéterminé.

9. Méthode de préparation d'une solution d'anticorps, consistant à faire une culture de cellules secrétant des anticorps à l'aide d'une méthode selon l'une quelconque des revendications 6 à 8.
